# EUROPEAN PATENT APPLICATION

(11) **EP 4 349 927 A1**
(43) Date of publication of application: **10.04.2024**
(21) Application number: 22306481.7
(22) Date of filing: 04.10.2022
(51) Int. Cl.: C09D 175/16, C08G 18/75, C08G 18/67, C08G 18/34, C08G 18/24, C08G 18/08, A61Q 3/04, A61Q 3/02, A61K 8/87, A61K 8/81

(54) **URETHANE (METH)ACRYLATES FOR USE IN RECYCLING OR DELAMINATION PROCESSES**

(71) Applicant: ARKEMA FRANCE, 92700 Colombes (FR)
(72) Inventor: SHURDHA, Endrit, EXTON, 19341 (US); HE, Yuhong, EXTON, 19341 (US); LI, Ling, EXTON, 19341 (US); MACNEILL, Christopher, EXTON, 19341 (US)
(74) Representative: Arkema Patent

(57) **Abstract**

A curable urethane (meth)acrylate oligomer is formed by reacting, in the presence of a catalyst, at least the following: (A) a first isocyanate reactive component bearing at least one (meth)acrylate group and at least one isocyanate reactive groups; (B) a polyisocyanate component; and (C) a second isocyanate reactive component bearing at least one acidic group and two isocyanate reactive groups, wherein the urethane (meth)acrylate oligomer has an acid content of at least 5×10⁻⁴ mol acid/g oligomer, with some of the acid groups optionally neutralized. A cured ink or coating made from the oligomer, along with at least one of a (meth)acrylate monomer or a (meth)acrylate oligomer and, optionally, at least one additive, can be easily removed from the substrate by treatment with aqueous base, thus allowing for recycling the substrate cleaned of any ink or coating. Nail gel compositions including the oligomer can also be more easily removed from nails.

## Description

### Field of the Invention

The invention relates to urethane (meth)acrylates, curable compositions based on the urethane (meth)acrylates, methods of using the urethane (meth)acrylates, and compositions and articles containing the urethane (meth)acrylates in cured form, especially for use in inks and coatings to allow for easy removal of the cured inks and coatings once subjected to recycling, soaking, or delaminating conditions.

### Background of the Invention

In view of the impacts of climate change and the increasing rate of consumption of our planet's natural resources, there has been a growing desire in the chemical industry to ensure sustainable consumption and production patterns. In the market segment of curable inks and coatings, this desire manifests itself in developing technologies that would permit easy and substantially complete delamination of the inks and coatings from their substrates. Such technologies would allow for separate recycling or reuse of the substrates. Efforts have been made to permit such separation and recycling, including by Siegwerk, which has recently introduced sacrificial deinking primers for labels and sleeves.

Another area of coatings that could benefit from improved ease of separation from its underlying substrate is the curable nail enamel segment. Curable nail coatings are applied to a consumer's bare nails to enhance their appearance and protect them from stresses of everyday life. Nail coatings are typically comprised of (meth)acrylate functionalized monomers, (meth)acrylate functionalized oligomers, non-reactive polymers, photoinitiator(s) (included for ultraviolet (UV)-curable nail coatings), and other additives, such as pigments and fillers, to give a 100% solids UV-curable nail formulation. Curable nail coatings other than UV-curable nail coatings are common and include peroxide cure coatings (including two-part cure coatings). Curable nail coatings (also referred to as enamel) may be applied in four coats - a base coat, two color coats, and a top coat. The base coat serves as an adhesive layer, while the color coat serves as a cosmetic layer. Finally, the top coat provides durability and scratch resistance and helps to prevent removal when a consumer comes in contact with certain chemical or physical stimuli. Once the nail coating is applied and cured under ultraviolet or LED light, the coating forms a durable, cross-linked network that can last up to two weeks. Nevertheless, typical UV-curable nail coatings can be difficult to remove and in some cases take up to 20 minutes with a solvent-based remover. In order to speed up the removability time, a nail technician may first abrade the surface of the nail polish with a coarse buffer to break the cross-linked film to allow for faster solvent penetration. This process can harm the nail plate and may leave the consumer's cuticles and nail surface feeling unpleasant and/or damaged.

Most literature that describes the removal of a paint coating or a pressure sensitive adhesive/laminating adhesive label uses either low vapor pressure or caustic-type solvent, both of which are non-biodegradable, harmful to the environment, and potentially harmful to the person using the solvent. See US2010008952A, WO2010115564A1. Neither of these removal processes are conducive for use in nail applications.

### Summary of the Invention

In view of the prior art, it would be desirable to develop a system which permits easy and substantially complete delamination of a curable coating or ink from a substrate without the need for a sacrificial primer layer. Furthermore, it would be advantageous to build a trigger in to the backbone of the UV curable coating, ink, or a nail enamel that can undergo removal in the presence of a non-hazardous, low VOC aqueous-based remover, but not in the presence of water alone. In many applicants, it would be undesirable for a coating or ink to delaminate from its substrate in the presence of water alone. Also, typical nail enamels should have high water resistance so that the polish is not removed during hand washing, swimming, in the shower, or washing dishes. Consequently, it would be desirable to provide a polish that can be removed on demand via an aqueous-based remover that can interact with the trigger in the backbone of the nail enamel and prompt the enamel's removal from the substrate.

The presence of the urethane (meth)acrylates (also referred to as acidic or acid-functional urethane (meth)acrylates or AUA's) of the invention in inks and coatings render such inks and coatings susceptible to removal from substrates to which they are attached when subjected to recycling conditions (or soaking conditions) described herein.

An aspect of the invention is a curable urethane (meth)acrylate oligomer having no free isocyanate groups at the terminal ends of the oligomer and formed by reacting, in the presence of a catalyst, at least the following:
(A) a first isocyanate reactive component bearing at least one (meth)acrylate group and at least one isocyanate reactive group;
(B) a polyisocyanate component; and
(C) a second isocyanate reactive component bearing at least one acidic group and two isocyanate reactive groups,
   wherein the urethane (meth)acrylate has an acid content of at least 5×10⁻⁴ mol acid/g oligomer.

Another aspect is an ink or coating composition comprising the urethane (meth)acrylate oligomer, a photoinitiator, and at least one of a (meth)acrylate monomer or a (meth)acrylate oligomer and, optionally, at least one additive.

Another aspect is a method for making an article comprising:
applying the ink or coating composition of the invention to a substrate; and
curing the ink or coating composition,
wherein the substrate is wood, plastic, metal, glass, stone, concrete, or a composite thereof.

Another aspect is a method for recycling a substrate coated with the cured ink or coating composition ink, wherein the method for recycling comprises:
contacting the article to which the cured ink or coating composition is attached with a recycling solution having a pH sufficient to delaminate the ink or coating composition from the substrate; and
retrieving from the recycling solution the substrate free from the ink or coating composition.

Another aspect is a nail gel composition comprising:
the urethane (meth)acrylate oligomer of the invention;
optionally, a photoinitiator;
at least one (meth)acrylate monomer or a (meth)acrylate oligomer; and optionally, at least one additive.

Nail gel compositions of the present invention intended for curing by actinic radiation, including UV radiation, include a photoinitiator. Nail gel compositions of the present invention intended for curing in some other manner, such as peroxide cure (including two-part curing), need not and typically do not contain a photoinitiator.

Another aspect is a method for coating a nail comprising:
applying the nail gel composition to a nail (either a human fingernail or toenail or a synthetic nail); and
curing the nail gel composition.

Another aspect is a method for removing the cured nail gel composition coated on the nail, wherein the method for removing the cured nail gel composition comprises:
immersing the cured nail gel composition coated on the nail with a soaking solution having a pH sufficient to partially or fully delaminate the cured nail gel composition from the nail; and
wherein, if the immersing step only partially delaminates the cured nail gel composition from the nail thereby leaving a portion of the cured nail gel composition attached to the nail, then the method further comprises manually removing from the nail the portion of the cured nail composition attached to the nail while the nail is still present in the soaking solution or, alternatively, after the nail is removed from the soaking solution.

It is common for a technician to use a tool to scrape the coating off of the nail. By permitting at least partial delamination of the coating, the invention permits for easier removal of the coating by providing for the technician a starting point for applying the tool. No chipping of the coating by the tool to initiate the removal is needed in accordance with this method of the invention.

### Brief Description of the Drawings

The following figures represent exemplary embodiments of the invention and are not intended to otherwise limit the description of the invention as described herein.
**Figure 1** shows a photograph of glass panels coated with control and coatings according to the invention after 20 minutes of soaking in water solution (pH = 5).
**Figure 2** shows a photograph of glass panels coated with control and coatings according to the invention after 20 minutes of soaking in a base solution (pH = 9).
**Figure 3** shows a photograph of glass panels coated with control and coatings according to the invention after 20 minutes of soaking in both water solution (pH = 5) and base solution (pH=9).

### Detailed Description of the Invention

### Definitions

As defined herein "acid content" is calculated as the total acid content of a compound (in moles) before any neutralization of the acid groups has taken place (i.e., assuming 100% of the acid groups are in their free acid (non-salt) form) per gram of the curable acid-functional urethane (meth)acrylate oligomers of the invention. When determining the acid content of a urethane (meth)acrylate oligomer that has at least some of its acid groups neutralized, it is presumed for purposes of this calculation that 100% of the acid groups exist in their free acid, regardless of whether the base(s) is/are provided: (1) during the preparation of the oligomer, (2) after the oligomer has been prepared but before formulation with other constituents, (3) after the oligomer has been formulated with other constituents one of which includes a base, and/or (4) in a recycling or soaking solution after the oligomer has been prepared, formulated, and cured. So, an acid-functional urethane (meth)acrylate oligomer of the invention having a certain acid content encompasses such an AUA that has none of the acid groups neutralized or at least some, including a majority and up to 100%, of its acid groups neutralized.

As defined herein, "acid group" refers to any functionality present in the curable urethane (meth)acrylate oligomers of the invention that contains an acidic hydrogen atom and primarily refers to carboxylic acid groups, but also includes functional groups such as sulfonic acids, phosphoric acids, phosphonic acids and phosphinic acids.

As defined herein, "aliphatic compound" or "aliphatic group" or "aliphatic linker" refers to a compound, group, or linker that is non-aromatic and acyclic, linear or branched, saturated or unsaturated, which may comprise one or more ether bonds, ester bonds, amide bonds, urethane bonds, urea bonds and mixtures thereof, and which may be substituted by one or more groups selected from, for example, alkyl, hydroxyl, halogen (F, Cl, Br, I), isocyanate, carbonyl, amine, carboxylic acid, -C(=O)-OR' and -C(=O)-O-C(=O)-R', with each R' being independently a C₁-C₆ alkyl.

As defined herein, "cycloaliphatic compound" or "cycloaliphatic group" or "cycloaliphatic linker" refers to a compound, group or linker that is non-aromatic and cyclic, which may comprise one or more aliphatic bonds, and which may be substituted by one or more aliphatic groups.

As defined herein, "C1-C5 group or compound" refers to a group or a compound having from 1 to 5 carbon atoms.

As defined herein, "nail" refers to a human fingernail or toenail and includes artificial extensions present on a fingernail or toenail.

As defined herein, a "recycling solution" or a "soaking solution" refers to a solution that when contacted with a composition (such a film or ink) containing an acidic urethane (meth)acrylate of the invention results in the release or delamination of the composition from a substrate to which the composition is attached. While the release or delamination of the acidic urethane (meth)acrylate-containing composition from the substrate to which it is attached is typically complete and total, a partial release or partial delamination of the composition may also represent an acceptable or satisfactory outcome, depending on the particular technical area involved, such as in the salon industry for nail treatments. In the case where delamination is only partial, manual means (such as a cuticle pusher tool or finger pressure or other known suitable means) may be employed for removal of the portion of the coating/composition that remains attached to the nail surface.

The curable acidic (also referred to herein as "acid-functional") urethane (meth)acrylate oligomers are prepared from components (A), (B), (C) and optionally (D) and/or (E) in the presence of a catalyst. The relative amounts of the components are driven by their stoichiometry to obtain a product as described herein, namely a difunctional urethane (meth)acrylate having no free isocyanate groups. More specifically, the second isocyanate reactive component bearing at least one acidic group and two isocyanate reactive groups (such as DMPA), the first isocyanate reactive component bearing at least one (meth)acrylate group (such as caprolactone acrylate), and the polyisocyanate component (such as IPDI) may be mixed in the presence of a catalyst and inhibitor and optionally with a solvent. The constituents are mixed, permitted to undergo the exothermic reaction, then heated for a temperature and time sufficient to reduce the free isocyanate to a *de minimis* amount. If the solvent was present, the solvent may then be stripped off and replaced with a monomer as process diluent. The catalyst is not particularly limited and may include tin-containing catalysts such as, but not limited to, tetrabutyltin, tetraoctyltin and tetraphenyltin. In at least one embodiment, the reaction mixture for forming the acidic urethane (meth)acrylates may also comprise other additives, such as, for example, inhibitors, surfactants, fillers, stabilizers, pigments, solvents, etc.

The acidic urethane (meth)acrylates of the invention may be used in curable compositions, preferably for coatings, adhesives, sealants, inks, 3D printing applications, electronics, composites, or cosmetics.

More particularly, the use of the acidic urethane (meth)acrylates of the invention relates to their presence in a curable composition which is a coating composition, more preferably for substrates that include human nails (in the salon industry), wood, plastic, metal, glass, fibers, textiles, leather, stone, ceramic, concrete or a composite.

### Component (A)

Component (A) of the acidic urethane (meth)acrylates of the invention represents a first isocyanate reactive component bearing at least one (meth)acrylate group and at least one isocyanate reactive group. The isocyanate reactive group is preferably a hydroxyl group. Suitable component (A) compounds include, but are not limited to, polyester (meth)acrylates, epoxy (meth)acrylates, polyether (meth)acrylates, urethane (meth)acrylates, hydroxy-bearing (meth)acrylates which in fact are partial (meth)acrylic esters of alkylene polyols which may be alkoxylated or partial (meth)acrylic esters of polyols resulting from di-polyol ethers of said alkylene polyols and combinations thereof. Examples of di-polyol ethers include di-trimethylol (diTMP) ether polyol (tetrol) or di-pentaerhythritol (diPE) ether polyol (hexol: 6 OH). The alkylene polyols or polyols correspond to di-alkylene polyol ethers can have from 2 to 6 hydroxy groups.

According to at least one embodiment, component (A) may comprise in addition to (meth)acrylate groups additional non-(meth)acrylate free-radical polymerizable functionalities such as allylic or vinylic groups.

In at least one embodiment, component (A) may comprise at least one (meth)acrylate functional group, in particular from 1 to 5 (meth)acrylate groups, more particularly at least 2 or 3 (meth)acrylate groups. In an embodiment, component (A) is a polyol component comprising a monoalcohol bearing at least 1, preferably from 1 to 5 (meth)acrylates groups. In another embodiment, component (A) is a diol bearing at least 1, preferably from 1 to 4, (meth)acrylate groups. For example, component (A) may comprise a monoalcohol of formula (1) bearing 3 (meth)acrylate groups or a diol of formula (2) bearing two (meth)acrylate groups, the formulas being shown below: 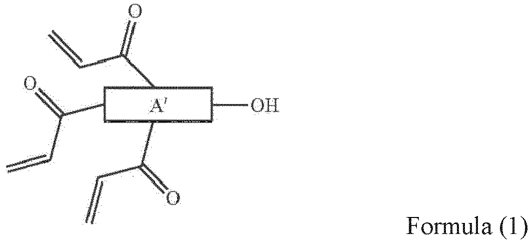 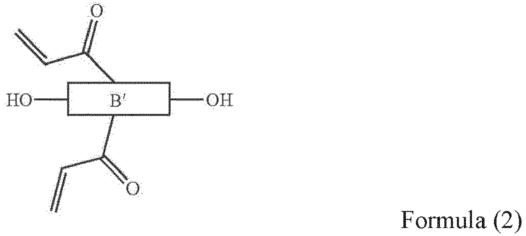 wherein A' and B' represent the residues of corresponding polyols (tetrols) partially esterified by acrylic acid and which may be linear, cyclic or branched, substituted or unsubstituted hydrocarbon chains, wherein the optional substituents include cyclic groups and/or heteroatoms. Chains A' and B' may, for example, comprise an ester or ether group.

Component (A) may be monomeric or oligomeric.

Suitable polyester (meth)acrylates include, but are not limited to, the reaction products acrylic or methacrylic acid or mixtures thereof with hydroxyl group terminated polyester polyols where the reaction process is conducted such that a significant concentration of residual hydroxyl groups remain in the polyester (meth)acrylate. The polyester polyols can be di-, tri-, tetra-, penta- or higher in hydroxyl group functionality.

The polyester polyols can be made by polycondensation reactions of di- or higher hydroxyl functional components with di- or higher functionality carboxylic acids or anhydrides. The hydroxyl functional and carboxylic acid components can each have linear, branched, cycloaliphatic or aromatic structures and can be used individually or as mixtures. Examples of suitable di-hydroxyl functional components include: 1,2-ethanediol, 1,2-propanediol, 1,3-propanediol, 1,4-butanediol, 1,5-pentanediol, 1,6-hexanediol, 1,10-decanediol, 1,12-dodecanediol, 1,3-butanediol, 2,2-dimethyl-1,3-propanediol, 2-methyl-1,3-propanediol, diethylene glycol, triethylene glycol, dipropylene glycol, tripropylene glycol, 1,4- and 1,6-dimethylolcylcohexane, C₃₆-dimer diol, hydroquinone bis(2-hydroxyethyl) ether (HQEE), hydroxypivaloyl pivalate and ethoxylated and/or propoxylated derivatives of the above. Ethoxylated and/or propoxylated derivatives of bisphenol A or bisphenol F are also suitable. Suitable tri- and higher hydroxyl functional components include: glycerol, trimethylolpropane, trimethylolethane, pentaerythritol, di-glycerol, di-trimethyolpropane, di-pentaerytritol, sorbitol and ethoxylated and/or propoxylated derivatives of the above. Examples of suitable di- or higher functional carboxylic acids include: malonic acid, succinic acid, maleic acid, fumaric acid, itaconic acid, glutaric acid, adipic acid, pimelic acid, sebacic acid, dodecanedioic acid, phthalic acid, isophthalic acid, terephthalic acid, naphthalene dicarboxylic acid, dimer fatty acids, trimellitic acid, pyromellitic acid and the anhydride derivatives of the above. Suitable polyester polyols can also be made by ring opening polymerization of lactones initiated by a hydroxyl functional starter molecule such as those described above. Suitable lactones include α,α-dimethyl-β-propiolactone, γ-butyrolactone and ε-caprolactone.

Examples of epoxy (meth)acrylates include the reaction products of acrylic or methacrylic acid or mixtures thereof with glycidyl ethers or esters. The glycidyl ethers or esters can have aliphatic, cycloaliphatic or aromatic structures and contain from two up to about six epoxy functional groups. Di-epoxy functional materials are preferred. Glycidyl ethers can be prepared from a hydroxyl functional precursor and an epoxy compound such as epichlorohydrin. Many of the hydroxyl functional components listed in the section above are suitable for preparation of aliphatic glycidyl ethers. Specific examples of precursors for aliphatic glycidyl ethers include: 1,4-butanediol, 2,2-dimethyl-1,3-propanediol, 1,6-hexanediol, 1,4- and 1,6-dimethylolcylcohexane, poly(ethylene glycol), poly(propylene glycol), poly(tetramethylene glycol), trimethylolpropane, pentaerythritol, glycerol and sorbitol. Specific examples of precursors for aromatic glycidyl ethers include: bisphenol A, bisphenol F and resorcinol.

Examples of suitable polyether (meth)acrylates include the condensation reaction products of acrylic or methacrylic acid or mixtures thereof with polyetherols which are polyether polyols. Suitable polyetherols can be linear or branched substances containing ether bonds and terminal hydroxyl groups. Polyetherols can be prepared by ring opening polymerization of cyclic ethers such as tetrahydrofuran or alkylene oxides with a starter molecule. Suitable starter molecules include water, the hydroxyl functional materials as described above, polyester polyols and amines. Examples of suitable amines include: ethylene diamine, 4,4'-diaminodiphenylmethane, diethylene triamine and hydroxyl amines such as ethanol amine and diethanol amine. Examples of suitable alkylene oxides include: ethylene oxide, propylene oxide, butylenes oxides, epichlorohydrin and glycidol. The polyether (meth)acrylates can be used individually or in combination.

Examples of polyurethane (meth)acrylates include the polyaddition products of the di- or polyisocyanates described below as component B with isocyanate reactive ethylenically unsaturated components as described in the sections above as polyester-, epoxy- or polyether (meth)acrylates or immediately below as monomeric hydroxyl (meth)acrylates.

Examples of monomeric hydroxyl (meth)acrylates are acrylic, methacrylic or mixed esters with simple diols, triols, tetrols or polyols where the esterification process is carried out such that residual hydroxyl groups remain in the final product. Examples include (meth)acrylate esters of: 1,2-ethanediol, 1,2-propanediol, 1,3-propanediol, 1,4-butanediol, 1,5-pentanediol, 1,6-hexanediol, 1,10-decanediol, 1,12-dodecanediol, 1,3-butanediol, 2,2-dimethyl-1,3-propanediol, 2-methyl-1,3-propanediol, diethylene glycol, triethylene glycol, dipropylene glycol, tripropylene glycol, 1,4- and 1,6-dimethylolcylcohexane, glycerol, trimethylolpropane, trimethylolethane, pentaerythritol, di-glycerol, di-trimethyolpropane, di-pentaerytritol and sorbitol. The monomeric hydroxyl (meth)acrylates can be used individually or in mixtures.

### Component (B)

Component (B) comprises a polyisocyanate component, including a diisocyanate. In at least one embodiment, component (B) may comprise a diisocyanate having two isocyanate functional groups, such as an aliphatic diisocyanate or a cycloaliphatic diisocyanate (e.g., isophorone diisocyanate). In other embodiments, component (B) may comprise a plurality of isocyanate groups, such as three or four or more isocyanate groups.

Non-limiting examples of compounds that may comprise component (B) include di- or polyisocyanates such as aliphatic, aromatic and cycloaliphatic structures with at least two isocyanate functional groups per molecule. Examples of suitable isocyanate components include: isophorone diisocyanate, hexamethylene diisocyanate, 2,3,3-trimethyl hexamethylene diisocyanate, 4,4'-dicyclohexylmethane diisocyanate, 1,5-naphthalene diisocyanate, 2,4- or 2,6-toluene diisocyanate and their isomeric mixtures, 4,4'-diphenylmethane diisocyanate. Polyisocyanates formed by creation of isocyanurate or biuret structures are also suitable. Mixtures of isocyanates are also suitable.

Preferably, the polyisocyanate is a cycloaliphatic diisocyanate having a cycloalkyl ring containing at least one C₁-C₃ alkyl substituent. More preferably, the polyisocyanate is isophorone diisocyanate (IPDI).

### Component (C)

Component (C) comprises a second isocyanate reactive component bearing at least one acidic group and two isocyanate reactive groups. Without wishing to be bound by theory, it is believed that component (C) aids in the solubilization of the oligomer in the composition. Therefore, in at least one embodiment, the composition may not comprise a surfactant. In other embodiments, a surfactant may be added to aid in the solubilization of the oligomer.

Component (C) is distinct from component (A). The at least one acidic group is preferably selected from a carboxylic acid group, a sulfonic acid group, a phosphoric acid group, a phosphonic acid group, a phosphinic acid group and salts thereof. The two isocyanate reactive groups are preferably hydroxyl groups.

In one embodiment, component (C) may comprise a polyol comprising an acidic group selected from carboxylic, sulfonic, phosphoric, phosphonic and phosphinic acid groups and salts thereof. In particular, component (C) may comprise a diol having a carboxylic or sulfonic acid group or a salt thereof.

Examples of component (C) include, but are not limited to, compounds containing at least two isocyanate reactive functional groups and at least one acidic group which can be ionic or potentially ionic in character. Combinations of the different types can be used. Ionic or potentially ionic groups include carboxylic acid, sulfonic acid or phosphoric acid groups or their alkali metal or quaternary amine salts. If the free acid forms are used to prepare oligomer, the acidic groups can be neutralized to the salt form before or during solubilization by addition of a base. Suitable bases include inorganic hydroxides or carbonates and amines and combinations. Specific examples of ionic/potentially ionic components with acidic nature include: hydroxyacetic acid, hydroxypropionic acid, malic acid, citric acid, dimethylolpropionic acid (DMPA), dimethylolbutanoic acid, 2-sulfo-1,4-butanediol, 2,5-dimethyl-3-sulfo-2,5-hexanediol, 2-aminoethanesulfonic acid, N-(1,1-dimethyl-2-hydroxyethyl)-3-amino-2-hydroxypropanesulfonic acid, 2-aminoethylaminoethanesulfonic acid and salts of the above.

According to a preferred embodiment, component (C) is a diol bearing an acidic group chosen from carboxylic acid, sulfonic acid, phosphoric acid, phosphonic acid or phosphinic acid groups, preferably carboxylic acid or sulfonic acid groups, more preferably a carboxylic acid group, with the group being at least partly neutralized with a basic agent.

According to a preferred embodiment, component (C) component has the following structure: 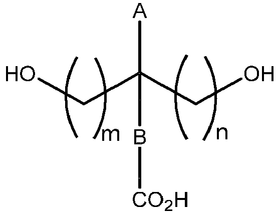 wherein A is hydrogen or a C₁-C₅ alkyl group; and B is a bond (i.e., nil) or a C₁-C₅ aliphatic linker; and m and n are independently 1, 2, 3 or 4, with the proviso that the component (C) is a C₄ to C₁₅ compound.

In a particular embodiment, component (C) comprises or is dimethyl propionic acid (DMPA).

### Component (D)

Optional component (D) is a base. Suitable inorganic bases include alkali metal and alkaline earth metal hydroxides, bicarbonates and carbonates (e.g., sodium hydroxide, potassium hydroxide, calcium hydroxide, sodium bicarbonate, potassium bicarbonate, sodium carbonate, potassium carbonate, calcium bicarbonate, calcium carbonate). Suitable organic bases include ammonia, pyridine and amines (e.g., isopropylamine, trimethylamine, diethylamine, triethylamine, tripropylamine, diethanolamine, ethanolamine, 2-dimethylaminoethanol, 2-diethylaminoethanol, dicyclohexylamine, etc.).

Component (D) also includes amine synergists.

The amount of component (D), if present, can vary from zero to an amount well in stoichiometric excess to that amount of base required to neutralize all of the acidic sites of the oligomer. One factor is whether the oligomer is expected to be exposed to a base (e.g., a solution comprising a base) either after formation of the oligomer but before curing or if a basic solution will be used as a recycling solution or soaking solution. The intended recycling and soaking conditions will also impact whether and the extent to which component (D) is included. Component (D) need not be included or may be included to a lesser extent with a more basic recycling or soaking solution and with higher recycling or soaking times and temperatures.

### Component (E)

Optional component (E) is an optional component in the acidic urethane (meth)acrylate curable compositions and may be a monalcohol or polyol. Component (E) is distinct from components (A) and (C). In embodiments of the acidic urethane (meth)acrylate curable compositions, component (E) is not present. If component (E) is present, it is not present in amounts sufficient to adversely impact the water solubility of the acidic urethane (meth)acrylate curable compositions.

In at least one embodiment, component (E) is a polyol. According to at least one embodiment, component (E) may be added to increase the molecular weight of the acidic urethane (meth)acrylate curable composition or to add additional functional groups to the acidic urethane (meth)acrylate curable composition.

Examples of component (E) include, but are not limited to, one or more polyols with from one to about six isocyanate reactive groups per molecule and molecular weight from about 200 to 5000 Daltons. Suitable polyols include polyesters, polyethers, polycarbonates, polycaprolactones, polybutadienes, hydrogenated polybutadienes, polyacrylics, polysiloxanes and fluorinated polyethers. Physical mixtures of the above or hybrid polyols with more than one structural type contained in the same molecule can be used.

According to at least one embodiment, component (E) may be monomeric or oligomeric.

Curing of the curable, acidic, urethane (meth)acrylate in accordance with the present invention may be carried out by any suitable method, such as free radical, thermal, electron beam, redox, Michael addition, cationic and/or anionic polymerization. One or more initiators, such as a free radical initiator (e.g., a photoinitiator, a peroxide initiator) may be present in the curable composition. In general, the curing step may comprise one of: (1) exposing the curable composition is to UV light or visible light; (2) exposing the curable composition to or e-an electron beam radiation; (3) initiating polymerization through the use of a redox-generated radical; or (4) initiating polymerization through the use of a thermally-generated radical. Curing may be accelerated or facilitated by supplying energy to the curable composition, such as by exposing the composition to a radiation source, such as visible light or UV energy, and/or electron beam radiation. In an exemplary embodiment, the curable urethane (meth)acrylate oligomer may be cured with UVC, UVB, UVA energy, and/or visible light. In an exemplary embodiment, the curable composition is cured with a UV light source utilizing UVA/UVB or UVA only and involves essentially no UVC radiation. In an exemplary embodiment, the curable composition is cured with high energy radiation, ranging from 0.01 to 10 W/cm². Possible light sources include, but are not limited to, natural outdoor light, black light, fluorescent light, or high pressure mercury light.

In an exemplary embodiment, the curing process is conducted by exposing the curable composition to ultraviolet radiation provided by one or more UV lamps delivering an irradiance level of 0.01 to 10 W/cm², such as 1 to 10 W/cm², for a time between 1 second and 30 minutes, such as between 1 second and 10 minutes. In another embodiment, the curing process is conducted by exposing the curable composition to e-beam radiation. In another embodiment, the curing process is conducted by exposing the curable composition to heat in the presence of a peroxide initiator (thermal curing). In another embodiment, the curing process occurs via redox polymerization which as a two-part process involving a peroxide initiator (e.g., as hydrogen peroxide, benzoyl peroxide or t-butyl hydroperoxide) as a first part and a reducing agent (e.g., a tertiary amine such as N,N-dimethylaniline, N-(4-methoxyphenyl)pyrrolidine and N-phenyldiethanol amine, sodium sulfite, sodium metabisulfite).

The curable urethane (meth)acrylate oligomer composition may further comprise one or more of additives that include, but are not limited to, antioxidants, ultraviolet absorbers, photostabilizers, foam inhibitors, solvents, flow or leveling agents, colorants, adhesion promoters, pigments, dispersants (wetting agents), slip additives, fillers, thixotropic agents, matting agents, thermoplastics such as acrylic resins that do not contain any free radical-polymerizable functional groups, waxes or other various additives, including any of the additives conventionally utilized in the coating, sealant, adhesive, molding or ink arts.

In certain embodiments of the invention (including curable compositions intended to be cured by actinic radiation, including UV radiation), the curable urethane (meth)acrylate oligomer compositions described herein include at least one photoinitiator. A photoinitiator may be considered any type of substance that, upon exposure to radiation (e.g., actinic radiation), forms species that initiate the reaction and curing of polymerizing organic substances present in the curable composition.

Free radical polymerization initiators are substances that form free radicals when irradiated. The use of free radical photoinitiators is especially preferred. Non-limiting types of free radical photoinitiators suitable for use in the curable compositions of the present invention include, for example, benzoins, benzoin ethers, acetophenones, benzyl, benzyl ketals, anthraquinones, phosphine oxides, α-hydroxyketones, phenylglyoxylates, α-aminoketones, benzophenones, thioxanthones, xanthones, acridine derivatives, phenazene derivatives, quinoxaline derivatives and triazine compounds.

Suitable photoinitiators include those capable of generating free radicals when exposed to the requisite radiation, such as UV light. In an exemplary embodiment, the photoinitiators include acyl phosphine oxides (*e.g*., Irgacure^{®} 819, Lucirin^{®} TPO and Lucirin^{®} TPO-L); benzil ketals (*e.g.,* Irgacure 651); alpha-hydroxy phenyl ketones *e.g.,* Irgacure 184 or Darocur 1173) or mixtures thereof.

The amount of photoinitiator may be varied as may be appropriate depending upon the photoinitiator(s) selected, the amounts and types of polymerizable species present in the curable composition, the radiation source and the radiation conditions used, among other factors. Typically, however, the amount of photoinitiator may be from 0.05% to 5%, preferably 0.1% to 2% by weight, based on the total weight of the curable composition.

Suitable solvents are any solvents that will dissolve all other components in the curable urethane (meth)acrylate oligomer composition and include aliphatic or aromatic hydrocarbons (*e.g.,* hexane, toluene or xylene), alcohols (*e.g.,* ethanol or propylene glycol), esters (*e.g.,* ethyl acetate, n-butyl acetate), ketones (*e.g.,* acetone, methyl isobutyl ketone or methyl ethyl ketone) and ethers (*e.g*., propylene glycol methyl ether or dimethoxyethane). In other embodiments, however, the curable compositions of the present invention may be formulated to be solvent-free, i.e., free of any non-reactive volatile substances (substances having a boiling point at atmospheric pressure of 150°C or less). For example, the curable urethane (meth)acrylate oligomer compositions of the present invention may contain little or no non-reactive solvent, *e.g*., less than 10% or less than 5% or less than 1% or even 0% of a non-reactive solvent, based on the total weight of the curable composition.

Any of the stabilizers known in the art related to (meth)acrylate-functionalized compounds may be utilized in the present invention. Quinones represent a particularly preferred type of stabilizer which can be employed in the context of the present invention. As used herein, the term "quinone" includes both quinones and hydroquinones as well as ethers thereof such as monoalkyl, monoaryl, monoaralkyl and bis(hydroxyalkyl) ethers of hydroquinones. Hydroquinone monomethyl ether is an example of a suitable stabilizer which can be utilized.

The concentration of stabilizer in the curable composition will vary depending upon the particular stabilizer or combination of stabilizers selected for use and also on the degree of stabilization desired and the susceptibility of components in the curable compositions towards degradation in the absence of stabilizer. Typically, however, the curable composition is formulated to comprise from 50 to 5000 ppm stabilizer.

Prior to curing, the curable urethane (meth)acrylate oligomer composition may be applied to a substrate surface in any known conventional manner, for example, by spraying, by brush, by sponge, by knife coating, by roller coating, by casting, by drum coating, by dipping, by curtain coating, by screen printing or by other methods of image transfer, by coating transfer, and the like and combinations thereof. Indirect application using a transfer process may also be used.

In an embodiment, the curable urethane (meth)acrylate oligomer composition may be applied directly to a substrate or over one or more of a primer, a basecoat system or other suitable layers in order to achieve the desired final appearance and properties. For example, the curable composition can be applied over a waterborne basecoat or a solvent-borne basecoat.

A substrate may be any commercially relevant substrate, such as a high surface energy substrate or a low surface energy substrate, such as a metal substrate or plastic substrate, respectively. The substrates may comprise metal, glass, plastics (e.g., thermoplastics such as polyolefins, polycarbonate, acrylonitrile butadiene styrene (ABS), and blends thereof), composites, wood, carbon fiberglass, nonwovens, ceramics, concrete and combinations thereof. Depending on the particular application, a suitable dry film thickness ranges from 5 to 200 microns. For 3D printing applications or electronics applications, the film may be thicker than 200 microns.

In an embodiment, the urethane (meth)acrylate oligomer has the following formula (II): 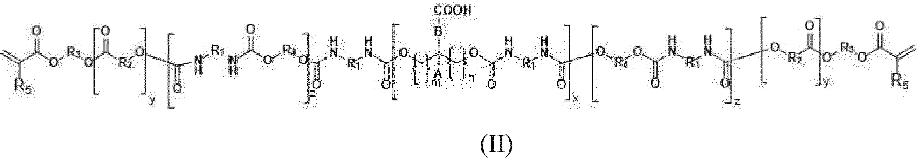 wherein:
each R₁, R₂, and R₃ is independently a C₁-C₁₂ aliphatic or cycloaliphatic linker;
R₄ is a residue of a polyol;
R₅ is either hydrogen or methyl;
A is hydrogen or a C₁-C₅ alkyl group;
B is a bond or a C₁-C₅ aliphatic linker; and
m and n are independently 1, 2, 3 or 4, with the proviso that the total number of carbon atoms in A, B, m, and n combined is an integer from 2 to 13; and
x and y are independently an integer from 0 to 5;
z is 0 or 1 to 10 and
optionally, at least a portion of the carboxylic acid groups exist in a salt form instead of a free acid form.

In a particular embodiment, the urethane (meth)acrylate oligomer has the following structure (III):

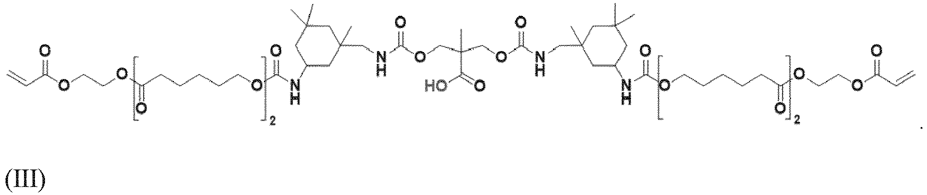

### Acid Content

The acid content of the curable urethane (meth)acrylate oligomers of the invention is at least 5×10⁻⁴ mol acid/g oligomer, preferably at least 5.25 ×10⁻⁴ mol acid/g oligomer, more preferably at least 5.5×10⁻⁴ mol acid/g oligomer, more preferably at least 6 ×10⁻⁴ mol acid/g oligomer, more preferably at least 7 ×10⁻⁴ mol acid/g oligomer, more preferably at least between 6 ×10⁻⁴ and 9.5×10⁻⁴ mol acid/g oligomer, such as between 6.5×10⁻⁴ and 9.25×10⁻⁴ mol acid/g oligomer, such as between 7×10⁻⁴ and 9×10⁻⁴ mol acid/g oligomer such as between 7.5×10⁻⁴ and 8.5×10⁻⁴ mol acid/g oligomer. In an embodiment, the upper limit of the acid content is not so high that the cured urethane (meth)acrylate oligomer undesirably easily delaminates in the presence of water. As used herein unless otherwise noted, the acid content refers to the number of moles of acid per gram of acidic (or acid-functional) urethane (meth)acrylate oligomers.

In the curable urethane (meth)acrylate oligomers of the invention, the percent of acid groups present in a free acid form (i.e., fully protonated) can range from 0% (i.e., all of the acid groups are in a neutralized (salt) form) to 100%. In various embodiments, preferably at least 5%, such as at least 10%, such as at least 15%, such as at least 20%, such as at least 25%, such as at least 30%, such as at least 35%, such as at least 40%, such as at least 45%, such as at least 50%, such as at least 55%, such as at least 60%, such as at least 65%, such as at least 70%, such as at least 75%, such as at least 80%, such as at least 85%, such as at least 90%, such as at least 95% of the acid groups neutralized (i.e., in a salt form). As mentioned above, this extent of neutralization will depend on a number of factors, including the expected recycling or soaking conditions.

In view of their acid content, the curable urethane (meth)acrylate oligomers of the invention are completely or substantially water soluble. This property readily distinguished these oligomers from previously reported polyurethane dispersions which are insoluble or substantially insoluble in water.

### Recycling/Soaking Solution

The recycling/soaking solution described herein causes release (delamination) of the cured acidic urethane (meth)acrylates of the invention (and the compositions, films, etc. in which they are present) from the substrates to which they are attached, resulting in effective recycling of the substrate.

In an exemplary embodiment, the recycling/soaking solution is an aqueous solution of a base (such as an inorganic base or an organic base). Other components may include, but are not limited to, surfactants and defoamers. The amount of the recycling/soaking solution used to effect release (delamination) of the cured acidic urethane (meth)acrylates is not particularly limited and may be present in a large stoichiometric excess relative to the cured acidic urethane (meth)acrylate.

In an exemplary embodiment, the pH of the recycling solution or soaking solution is basic - i.e., has a pH greater than 7. In an exemplary embodiment, the pH is greater than 7 and less than 13, such as greater than 7 and less than 12, such as greater than 7 and less than 11, such as greater than 7 and less than 10, such as greater than 7 and less than 9. The pH will depend on the extent of neutralization of the free acid groups of the urethane (meth)acrylate backbone by having reacted such free acid groups with a base already. With increasing prior neutralization, a recycling solution or soaking solution with a lower pH can be used and have the same effect as a recycling solution or soaking solution having a higher pH on a composition having a urethane (meth)acrylate backbone having a lower extent of neutralization.

As used herein, the use of the phrase "soaking solution" typically refers to a solution that is in contact with human skin via fingers or toes containing a natural human nail or an artificial nail (i.e., the human nail or the artificial nail is the substrate). As a result, the components of the soaking solution must be compatible with exposure to human skin over a period of time sufficient for release of the coating that is present on the nail surface. In an exemplary embodiment, the maximum pH of the soaking solution is 11 or less, such as 10 or less, such as 9 or less, such as 8 or less, such as greater than 7 and less than 9, such as greater than 7 and less than 8.

Suitable bases for inclusion in the soaking solution are not particularly limited and include inorganic bases and organic bases. Suitable inorganic bases include alkali metal and alkaline earth metal bicarbonates or carbonates (e.g., sodium bicarbonate, potassium bicarbonate, sodium carbonate, potassium carbonate, calcium bicarbonate, calcium carbonate). Inorganic bases such as alkali metal and alkaline earth metal hydroxides (e.g., sodium hydroxide, potassium hydroxide, calcium hydroxide) are typically too caustic for inclusion in a soaking solution that will contact human skin unless present in dilute amounts (e.g., 2% by weight or less in water). Suitable organic bases include ammonia, pyridine and amines (e.g., isopropylamine, trimethylamine, diethylamine, triethylamine, tripropylamine, diethanolamine, ethanolamine, 2-dimethylaminoethanol, 2-diethylaminoethanol, dicyclohexylamine, etc.).

Curing of the curable (meth)acrylate-containing composition in accordance with the present invention may be carried out by any suitable method, such as free radical, cationic and/or anionic polymerization. One or more initiators, such as a free radical initiator (e.g., a photoinitiator, a peroxide initiator) may be present in the curable composition. In general, the curing step may comprise one of: (1) exposing the curable composition is to UV light or visible light; (2) exposing the curable composition to or e-an electron beam radiation; (3) initiating polymerization through the use of a redox-generated radical; or (4) initiating polymerization through the use of a thermally-generated radical.

Curing may be accelerated or facilitated by supplying energy to the curable composition, such as by exposing the composition to a radiation source, such as visible light or UV energy, and/or electron beam radiation. Thus, the cured composition may be deemed to be the reaction product of the curable composition, formed by curing.

The curable (meth)acrylate-containing compositions of the present invention are particularly well suited to being cured using LED (Light Emitting Diode) curing (e.g., UV LED curing, using radiation from a UV LED device) and for use in high speed applications (such as coatings). Ease of delamination is dependent on the ease with which the recycling solution is able to permeate the coating or ink or film composition containing the cured urethane (meth)acrylate oligomer, which is impacted by physical properties of the composition, including the degree of cross-linking, glass transition temperature (Tg), and its hydrophilicity. Triggered removability of these coatings is influenced by four main conditions 1) crosslink density of the composition 2) number of triggered functional groups within the composition 3) removal time and 4) removal temperature. The crosslink density is important because if the composition is too highly crosslinked, the remover will not be able to penetrate through the film and trigger removability via a swelling mechanism. In addition, there has to be a certain number of acidic functional groups present in the film that can allow for enough swelling. The removal time is important because it allows the aqueous based remover to penetrate the film and temperature of the remover during removal can help with softening of the film to increase penetration and mobility. Regardless of the ease of removability of a composition containing a urethane acrylate of the present invention, the inclusion of the urethane acrylate of the invention in such a composition has been found to make such removability easier.

Without attempting to assess the percentage of acid groups that are in free acid form versus the percentage of acid groups that are in a neutralized form, the following test conditions may be used, in one aspect of the invention, as a standard for achieving complete delamination of a coating or film or ink or other composition containing the cured urethane (meth)acrylate oligomer from the substrate to which the coating or film or ink or other composition is attached: Upon exposure of the coated substrate to a 2% sodium hydroxide (NaOH) solution at 85°C for 10 minutes, complete delamination of the coating from the substrate occurs. Utilizing the urethane acrylate of the present invention can achieve this in certain formulations (e.g., ones that do not increase the cross link density and utilize a sufficient amount of the urethane acrylate of the invention) and in other formulations, utilizing the urethane acrylate of the invention increases the extent of delamination under such conditions compared to a similar formulation that does not contain a urethane acrylate of the present invention.

### Aspects of the Invention

Illustrative non-limiting embodiments of the present invention may be summarized as follows:
Aspect 1: A curable urethane (meth)acrylate oligomer having no free isocyanate groups at the terminal ends of the oligomer and formed by reacting, in the presence of a catalyst, at least the following:
   (A) a first isocyanate reactive component bearing at least one (meth)acrylate group and at least one isocyanate reactive group;
   (B) a polyisocyanate component; and
   (C) a second isocyanate reactive component bearing at least one acidic group and two isocyanate reactive groups,
      wherein the urethane (meth)acrylate has an acid content of at least 5×10⁻⁴ mol acid/g oligomer, such as at least 5.25 ×10⁻⁴, such as at least 5.5×10⁻⁴, preferably between 6 ×10⁻⁴ and 9.5×10⁻⁴, more preferably between 6.5×10⁻⁴ and 9.25×10⁻⁴, more preferably between 7×10⁻⁴ and 9×10⁻⁴ and most preferably between 7.5×10⁻⁴ and 8.5×10⁻⁴ mol acid/g oligomer.
Aspect 2: The urethane (meth)acrylate oligomer of Aspect 1, wherein the two isocyanate reactive groups of the component (C) are hydroxyl groups and wherein the at least one acidic group of the component (C) is selected from selected from a carboxylic acid group, a sulfonic acid group, a phosphoric acid group, a phosphonic acid group, a phosphinic acid group and salts thereof.
Aspect 3. The urethane (meth)acrylate oligomer of Aspect 1 or Aspect 2, wherein the percent of acid groups present in a free acid form (i.e., fully protonated) is 0% (i.e., all of the acid groups are in a neutralized (salt) form) to 100%.
Aspect 4. The urethane (meth)acrylate oligomer of any of Aspects 1-3, wherein the percent of acid groups present in a free acid form is at least 5%, such as at least 10%, such as at least 15%, such as at least 20%, such as at least 25%, such as at least 30%, such as at least 35%, such as at least 40%, such as at least 45%, such as at least 50%, such as at least 55%, such as at least 60%, such as at least 65%, such as at least 70%, such as at least 75%, such as at least 80%, such as at least 85%, such as at least 90%, such as at least 95%.
Aspect 5. The urethane (meth)acrylate oligomer of any of Aspects 1-4, wherein the percent of acid groups present in a neutralized (salt) form is at least 5%, such as at least 10%, such as at least 15%, such as at least 20%, such as at least 25%, such as at least 30%, such as at least 35%, such as at least 40%, such as at least 45%, such as at least 50%, such as at least 55%, such as at least 60%, such as at least 65%, such as at least 70%, such as at least 75%, such as at least 80%, such as at least 85%, such as at least 90%, such as at least 95%.
Aspect 6: The urethane (meth)acrylate oligomer of any of Aspects 1-5, wherein the component (C) has the following structure: 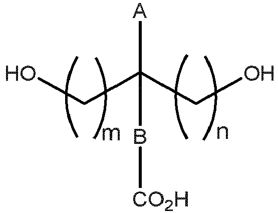 wherein: A is hydrogen or a C₁-C₅ alkyl group; and B is a bond or a C₁-C₅ aliphatic linker; and m and n are independently 1, 2, 3 or 4, with the proviso that the component (C) is a C₄ to C₁₅ compound.
Aspect 7: The urethane (meth)acrylate oligomer of any of Aspects 1-6, wherein the component (C) is dimethyl propionic acid (DMPA).
Aspect 8: The urethane (meth)acrylate oligomer of any of Aspects 1-7 wherein the component (A) comprises a lactone (meth)acrylate selected from the group consisting of β-propiolactone (meth)acrylate, γ-butyrolactone (meth)acrylate, δ-valerolactone (meth)acrylate and ε-caprolactone (meth)acrylate), and preferably is caprolactone acrylate.
Aspect 9: The urethane (meth)acrylate oligomer of any of Aspects 1-8, wherein the polyisocyanate is a cycloaliphatic diisocyanate having a cycloalkyl ring containing at least one C₁-C₃ alkyl substituent.
Aspect 10: The urethane (meth)acrylate oligomer of any of Aspects 1-9, wherein the polyisocyanate is isophorone diisocyanate (IPDI).
Aspect 11: The urethane (meth)acrylate oligomer of any of Aspects 1-10, wherein the oligomer is formed by reacting a base (D) with components (A), (B), and (C) or alternatively, subsequent to reaction of components (A), (B), and (C), in an amount such that a majority of the acid groups present in the oligomer are in a salt form instead of a free acid form.
Aspect 12: The urethane (meth)acrylate oligomer of any of Aspects 1-11, wherein the oligomer is formed by reacting a polyol (E) with components (A), (B), and (C) and optionally a base (D).
Aspect 13: The urethane (meth)acrylate oligomer of any of Aspects 1-11, wherein the oligomer does not contain any polyol other than component (C).
Aspect 14: The urethane (meth)acrylate oligomer of any of Aspects 1-12, having the formula (II): 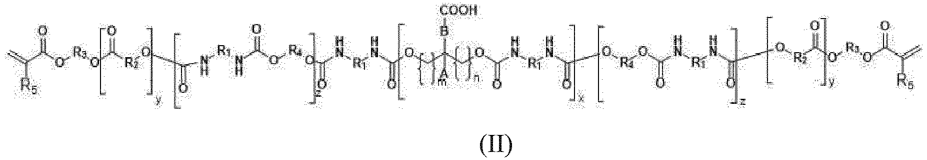 wherein:
   each R₁, R₂, and R₃ is independently a C₁-C₁₂ aliphatic or cycloaliphatic linker;
   R₄ is a residue of a polyol;
   R₅ is either hydrogen or methyl;
   A is hydrogen or a C₁-C₅ alkyl group;
   B is a bond or a C₁-C₅ aliphatic linker; and
   m and n are independently 1, 2, 3 or 4, with the proviso that the total number of carbon atoms in A, B, m, and n combined is an integer from 2 to 13; and
   x and y are independently an integer from 0 to 5;
   z is 0 or 1 to 10 and
   optionally, at least a portion of the carboxylic acid groups exist in a salt form instead of a free acid form.
Aspect 15. The urethane (meth)acrylate oligomer of any of Aspects 1-12, having the formula (III): 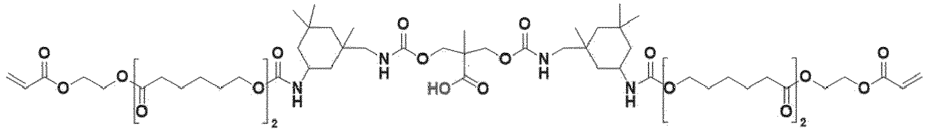
Aspect 16: The urethane (meth)acrylate oligomer of any of Aspects 1-15, wherein the catalyst is a tin-containing catalyst.
Aspect 17: A film or coating composition comprising the urethane (meth)acrylate oligomer of any of Aspects 1-16 and a photoinitiator.
Aspect 18. An ink comprising the urethane (meth)acrylate oligomer of any of Aspects 1-16 and a photoinitiator.
Aspect 19. A method for making an article comprising: applying the film or coating composition of Aspect 17 to a substrate; and curing the film or coating composition, wherein the substrate is metal, glass, plastic (e.g., thermoplastics such as polyolefins, polycarbonate, acrylonitrile butadiene styrene (ABS), and blends thereof), composites, wood, carbon fiberglass, nonwovens, ceramics, concrete, and composites thereof.
Aspect 20. The method of Aspect 18, wherein the substrate is plastic.
Aspect 21. A method for making an article comprising: applying the ink of Aspect 18 to a substrate; and curing the ink, wherein the substrate is metal, glass, plastic (e.g., thermoplastics such as polyolefins, polycarbonate, acrylonitrile butadiene styrene (ABS), and blends thereof), composites, wood, carbon fiberglass, nonwovens, ceramics, concrete, and composites thereof.
Aspect 22. The method of Aspect 21, wherein the substrate is plastic.
Aspect 23. A method for recycling a substrate coated with the cured film or cured coating composition according to Aspect 19, wherein the method for recycling comprises: contacting the article to which the cured film or coating composition is attached with a recycling solution having a pH sufficient to delaminate the film or coating composition from the substrate; and retrieving from the recycling solution the substrate free from the film or coating composition.
Aspect 24. The method of Aspect 23, wherein the substrate is plastic.
Aspect 25. The method of Aspect 23 or Aspect 24, wherein the pH of the recycling solution is ≥7, such as greater than 7 and less than 13, such as greater than 7 and less than 12, such as greater than 7 and less than 11, such as greater than 7 and less than 10, such as greater than 7 and less than 9.
Aspect 26. The method of any of Aspects 23-25, wherein delamination occurs within 10 minutes of exposure to the recycling solution.
Aspect 27. The method of any of Aspects 23-25, wherein when the pH of the recycling solution corresponds to a 2% NaOH solution and delamination occurs within 10 minutes of exposure at 85°C.
Aspect 28. The method of any of Aspects 23-27, wherein the base is an inorganic base selected from the group consisting of sodium hydroxide, potassium hydroxide, sodium carbonate, potassium carbonate, sodium bicarbonate, and potassium bicarbonate.
Aspect 29. The method of any of Aspects 23-28, wherein the base is an organic base that is an amine.
Aspect 30. A method for recycling a substrate coated with the cured ink according to Aspect 21, wherein the method for recycling comprises: contacting the article to which the cured ink is attached with a recycling solution having a pH sufficient to delaminate the ink from the substrate; and retrieving from the recycling solution the substrate free from the ink.
Aspect 31. The method of Aspect 30, wherein the substrate is plastic.
Aspect 32. The method of Aspect 30 or Aspect 31, wherein the pH of the recycling solution is ≥7, such as greater than 7 and less than 13, such as greater than 7 and less than 12, such as greater than 7 and less than 11, such as greater than 7 and less than 10, such as greater than 7 and less than 9.
Aspect 33. The method of any of Aspects 30-32, wherein delamination occurs within 10 minutes of exposure to the recycling solution.
Aspect 34. The method of any of Aspects 30-33, wherein when the pH of the recycling solution corresponds to a 2% NaOH solution and delamination occurs within 10 minutes of exposure at 85°C.
Aspect 35. The method of any of Aspects 30-34, wherein the base is an inorganic base selected from the group consisting of sodium hydroxide, potassium hydroxide, sodium carbonate, potassium carbonate, sodium bicarbonate, and potassium bicarbonate.
Aspect 36. The method of any of Aspects 30-35, wherein the base is an organic base that is an amine.
Aspect 37. A nail gel composition comprising: the urethane (meth)acrylate oligomer according of any of Aspects 1-16; at least one of a (meth)acrylate monomer or a (meth)acrylate oligomer; and optionally, at least one additive.
Aspect 38. A method for coating a nail comprising: applying the nail gel composition of Aspect 37 to a nail; and curing the nail gel composition.
Aspect 39. A method for removing the cured nail gel composition coated on the nail according to the method of Aspect 38, wherein the method for removing the cured nail gel composition comprises: immersing the cured nail gel composition coated on the nail with a soaking solution having a pH sufficient to partially or fully delaminate the cured nail gel composition from the nail; wherein, if the immersing step only partially delaminates the cured nail composition from the nail thereby leaving a portion of the cured nail composition from the nail attached to the nail, then the method further comprises manually removing from the nail the portion of the cured nail composition attached to the nail while the nail is still present in the soaking solution or, alternatively, after the nail is removed from the soaking solution..
Aspect 40. The method of Aspect 38 or Aspect 39, wherein the pH of the soaking solution is ≥7.
Aspect 41. The method of any of Aspects 38-40, wherein the pH of the soaking solution is ≥7 such as greater than 7 and less than 11, such as less than 10, such as less than 9, such as greater than 7 and less than 8.
Aspect 42. The method of any of Aspects 38-41, wherein the soaking solution comprises a base selected from an inorganic base or an organic base.
Aspect 43. The method of any of Aspects 38-42, wherein the base is an inorganic base selected from the group consisting of sodium carbonate, potassium carbonate, sodium bicarbonate, and potassium bicarbonate.

### Examples

### Example 1. Acidic Urethane (Meth)acrylate

1 mole dimethylol propionic acid (DMPA), 1.8 moles SR495B (caprolactone acrylate), 2 moles isophorone diisocyanate (IPDI), 0.2 wt% BHT as inhibitor, 0.09 wt% dibutyl tin dilaurate (DBTDL) as catalyst and 20 wt% cyclopentanone as process solvent were mixed well with air sparge and allowed exothermic temperature to reach 65C then heated to 110C until all DMPA solids disappeared and intermediate NCO stalled (< 0.5% by FTIR). The remaining 0.2 mole SR495B was then added in and the reaction was run at 110C until NCO% < 0.1% by FTIR (DMPA interfered NCO% titration). Cyclopentanone was stripped off at 100C/10 mmHg with agitation. The final product having the structure shown as structure (III) had an acid content of 0.0008 mol/gm.

### Example 2. Acidic Urethane (Meth)acrylate

1 mole dimethylol propionic acid (DMPA), 1.6 moles PRO13061 (higher molecular weight caprolactone acrylate), 2 moles isophorone diisocyanate (IPDI), 0.2 wt% BHT as inhibitor and 0.09 wt% DBTDL as catalyst were mixed well with air sparge and allowed exothermic temperature to reach 100C then heated to 110C until all DMPA solids disappeared and intermediate NCO stalled (< 1.2% by FTIR). The remaining 0.4 mole PRO13061 was then added in and the reaction was run at 110C until NCO% < 0.1% by FTIR (DMPA interfered NCO% titration). No process solvent or monomer diluent was needed in this reaction due to lower viscosity of the neat product. The final product having the structure shown as structure (IV) had final product had an acid content of 0.0006 mol/gm.

### Comparative Example 3. Acidic Urethane (Meth)acrylate

1.3 moles dimethylol propionic acid (DMPA), 6.5 moles isophorone diisocyanate (IPDI), 0.2 wt% Irganox 1035 (BASF) as inhibitor, 0.06 wt% Reaxis C716 (a bismuth carboxylate) as catalyst and 10 wt% acetone as process solvent were mixed well with air sparge and heated to 78°C then held at 78°C with agitation until all DMPA disappeared. 1 mole poly(neopentyl adipate) glycol polyester polyol having MW of 500Da (Fomrez^{®} 55-225), 1 mole CN104Z (Sartomer, epoxy acrylate) and 1.1 moles SR356 (Sartomer, acrylate ester) were added into the mixture and allowed exothermic temperature to reach 80°C. The reaction was run at 80C until intermediate NCO < 5.5% by FTIR. 5.4 moles methanol was then added in and the reaction was run at 80C until NCO% < 0.1% by FTIR. Acetone was then stripped off at 80C/10 mmHg with agitation. The final product had an acid content of 0.0004 mol/gm. The urethane acrylate of this Comparative Example 3 did not solubilize in basic water solution. Its solubility differs greatly compare to the newly proposed acidic urethane (meth)acrylate of Examples 1 and 2 and cannot be utilized in the removability applications discussed herein.

### Example 4. Neat Properties of Acidic Urethane (Meth)acrylate

5 gr of acidic urethane (meth)acrylate (AUA) of Example 1 was mixed with 5 gr of deionized water. 0.5 gr of 25% NaOH solution was added slowly to neutralize the acid groups. Upon sonication, the mixture became homogeneous as the AUA neutralized and dissolved in water. 4% of Speedcure KEM photoinitiator was added to the precious mixture and blended together. A 0.1-0.2 mm thickness film was prepared on an aluminum panel and placed in a 65°C oven for 2 minutes to evaporate the water and form a liquid acrylate film. At this stage, the dried liquid film was UV cured in the Impro unit with 400W/in² at 50 ft/min. The cured film was hard and very difficult to scratch from the aluminum panel. This aluminum panel was placed in water and in slightly over 2 minutes, the water-exposed part of the film absorbed water, swelled, and delaminated from the aluminum panel. The swollen film was very soft and easily broken.

### Example 5. Formulated Properties of Acidic Urethane (Meth)acrylate

The acidic urethane (meth)acrylate (AUA) of Example 1 was blended at equivalent weight with SR9003B, which is propoxylated neopentyl glycol diacrylate, before and after the neutralization step of Example 4, and 4% PL 460 photoinitiator. The two different mixtures, one having the unneutralized AUA of Example 1 and one having the neutralized AUA of Examples 1 and 4, were printed on a PET flexible film with a thickness of 0.02-0.05 mm using Impro curing unit with 400w/in2 at 50ft/m. The cured films were cut into strips and placed in a 2% NaOH solution at 80-85C while stirring. The films were in the base solution for 10 min.

The formulated acidic (i.e., unneutralized) urethane (meth)acrylate showed signs of delamination but not fully removed from the PET film. Standing overnight in the base bath at room temperature did show some swelling and overall delamination. This film behavior can be explained by slow neutralization of the acid groups followed by swelling of the film. It is expected that using a more basic solution would shorten the time of delamination.

The formulated neutralized acidic urethane (meth)acrylate film did swell and delaminate from the PET film within two minutes of exposure on the base bath. The cured acrylate film did swell and disintegrated into smaller fragments while the base solution was stirring. This film weakening was expected as the original neutralized acidic urethane (meth)acrylate swelled and weakened upon exposure to water.

### Example 6. Formulated Acidic Urethane (Meth)acrylate with Amine Synergist

The formulated, neutralized acidic urethane (meth)acrylate of Example 5 was blended with CN383, an acrylated amine synergist, at a 1 to 1 mole ratio to neutralize the acid groups with a tertiary amine. The IR data indicates a shift and reduction of the carbonyl peak of the carbonic acid when neutralized with the tertiary amine. The blend was mixed with 4% PL460 and a 0.02-0.05 mm thick film was prepared on PET substrate. The film was cured with the Inpro Technologies mercury arc curing unit with 400W/in2 at 50ft/min. The cured film was placed in the deinking conditions with the base bath for 10 min similar to previous examples. The cured film did delaminate and disintegrate within 3 min. This film formulation did show a slight delay of 30-60s when compared to the inorganic neutralized film.

### Example 7 Coating Compositions using Acidic Urethane Meth(acrylate) Oligomer

Coating compositions, which may be suitable as UV-curable nail gel formulations, were made by adding a primary oligomer (CN1968 or acidic CN1968, both of which are difunctional urethane (meth)acrylate oligomers), monofunctional monomer (CN147MA), and photoinitiator (TPO-1) along with either of the acidic urethane acrylates (III) (shown above) or (IV), shown below. The compositions, as formulated, had acid values of 5 mg KOH/g resin and 90 mg KOH/g resin, respectively, in accordance with ASTM D 974. The acid values of the formulations were measured using a Mettler Toledo G20S AutoTitrator. 0.5 g - 5 g of formulation was added to a 125 mL plastic beaker according to expected acid value. Acetone (30 mL) was added and the solution was mixed to dissolve the formulation. Water (1 mL) was added and mixed for 2 minutes. The stir bar was removed and the electrodes were immersed in the beaker. Acid value was measured using a LabX software package. 0.1 N NaOH as titrant was added to the solution by the 'AutoTitrator," and an inflection point was visible after titration. The derivative of the pH curve was measured and corresponds to a particular acid value, which was recorded. The acid contents of AUA (III) and AUA (IV) remained at 0.0008 mol/gm and 0.0006 mol/gm, respectively. 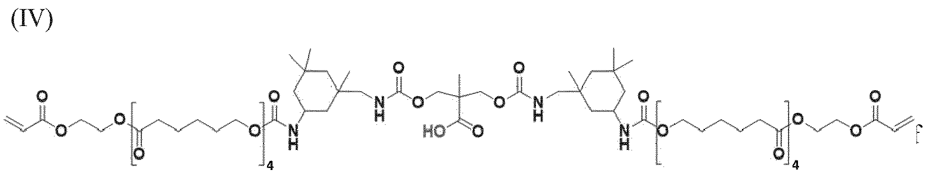 AUA (III). The components were added to a Flacktek polypropylene cup and mixed until homogenous at 2000 rpm for 2 minutes using a Flacktek^{®} DAC 400.2 VAC high speed mixer.

To prepare curable UV coatings compositions (which are suitable as nail gel compositions), a sample was pipetted onto a clean glass panel (4 inch × 4 inch). A ByK 3 Mil draw down bar was used to draw down the sample onto the glass. The coating was placed in a Gelish 18G LED nail lamp for 60 seconds. Once cured, initial color, haze and yellowness of the films were measured using a Hunter Lab ColorQuest XE spectrometer with glass as reference. Other performance properties such as surface tack, Konig hardness, and adhesion were also measured. Konig hardness of the coatings formulations were measured using ASTM D4366. Surface tack was determined qualitatively by assigning a number from the arbitrary scale between 0-5 (0 for no tack/ 5 for very tacky).

Once cured, the glass panel with coating was immersed in either water or 5% sodium bicarbonate solution for an allotted period of time (5 min, 10 min, 15 min, 20 min). The coated panels were removed and swellability/removability was measured visually.

UV curable coating formulations, which are suitable as nail gel formulations, were made containing a 25% loading of each acidic urethane acrylate oligomer (III) and acidic urethane acrylate (IV) with 47% loading of two different primary oligomers, CN1968 and an acidic version of CN1968 that contains DMPA acid attached to the backbone. All formulations contained 25% loading of a monofunctional monomer diluent (CN147MA) and 3% loading of photoinitiator (TPO-1). The formulations were mixed, drawn down on glass, and cured using a Gelish 18G LED nail lamp. The performance properties were tested and are shown in the table below.

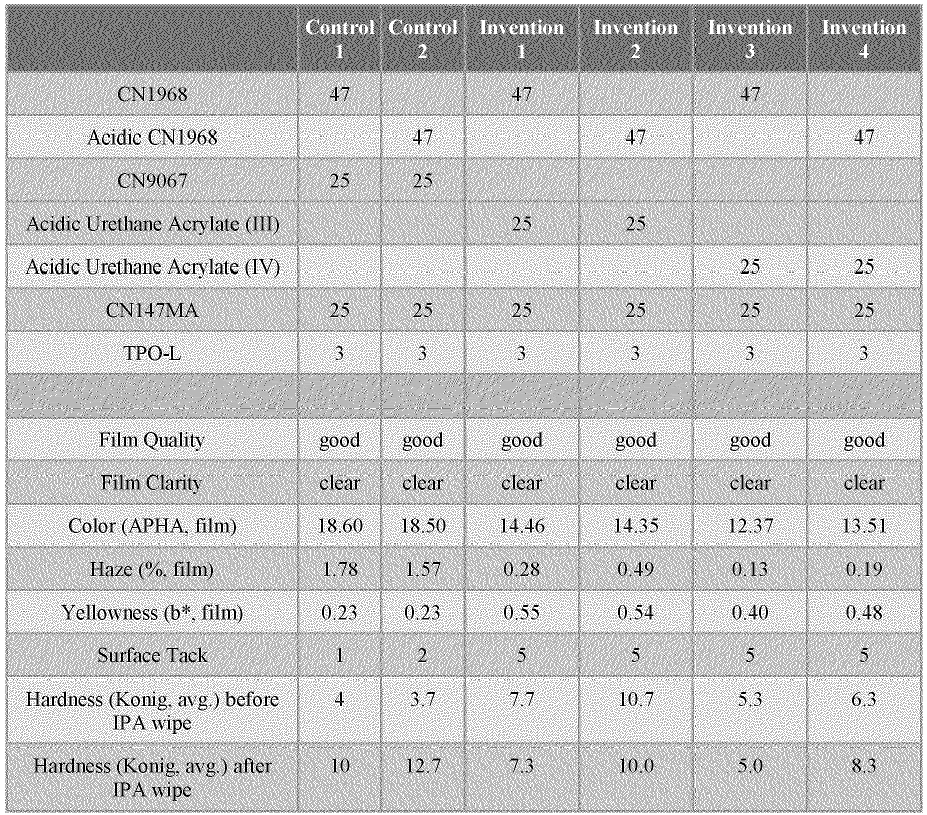

The performance properties of the nail gel compositions containing the acidic urethane acrylates (III) and (IV) were very similar to the performance of the control formulations. The initial color (APHA, 10 mm) and yellowness (b^{∗}) of the films after photocuring for both the invention and control samples were very low. Initial haze was lower for the invention samples compared to the control samples. Konig hardness was similar for all samples before and after IPA wipe.

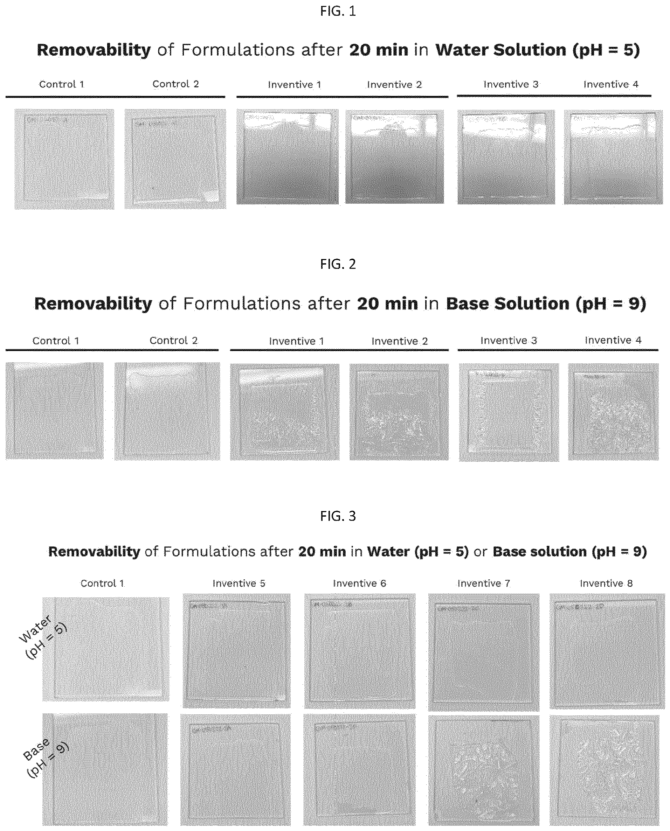

In order to determine whether the compositions according to the invention offered a triggered removal process but did not delaminate too readily in merely water (which would be undesirable), the control samples were compared to the compositions according to the invention for removability in water solution (pH = 5). Figure 1 shows a picture of each of the glass panels with the nail coating applied after 20 minutes of soaking in water solution (pH = 5). Figure 1 reveals that all of the coatings remained intact and adhered to the glass panel after 20 minutes of water soak time. The removability of the nail compositions in sodium bicarbonate base solution (pH = 9) for 20 minutes at room temperature was also tested. Figure 2 shows a picture of each of the glass panels with the nail coating applied after 20 minutes of soaking in base solution (pH = 9). Figure 2 reveals that the coating remains intact and adhered to the glass panel after 20 minutes of soaking for the Control 1 and Control 2 compositions, each of which do not contain an acidic urethane acrylate. For invention compositions 1, 2, 3, and 4 (all of which contain 25% of either acidic urethane acrylate III or IV), the films began to remove from the glass surface and caused excessive wrinkling due to swelling. Swelling was more apparent in Invention 2 and Invention 4 vs. Invention 1 and Invention 3 due to the addition of the acidic CN1968, which helped improve removability. The basic water solution was able to penetrate the film and cause the film to swell and delaminate from the glass panel. This only occurs in a basic water solution with pH = 9, but not in water solution (pH =5), which is important for consumers.

Next, an assessment of the amount of the acidic urethane acrylates III and IV that is needed to induce removability in base solution (pH = 9) was made. We chose to use only CN1968 as the primary oligomer and not the acidic CN1968. UV curable formulations, suitable as nail gel formulations, containing a 14.3% and 33.3% loading of each acidic urethane acrylate oligomer (III) and acidic urethane acrylate (IV) with between 42 and 54% loading of CN1968 were made. Again, all formulations contained 22-29% loading of a monofunctional monomer diluent (CN147MA) and around 3% loading of photoinitiator (TPO-1). The formulations were mixed, drawn down on glass, and cured using a Gelish 18G LED nail lamp. The performance properties were tested and are shown in the table below.

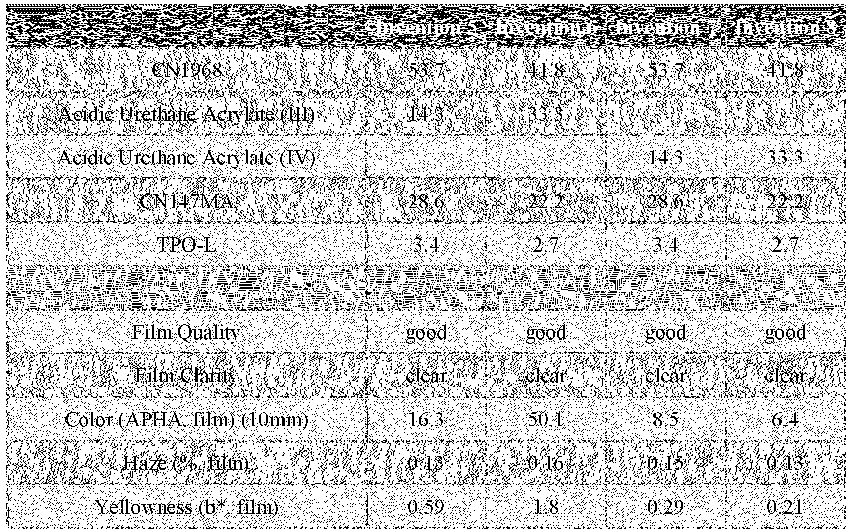

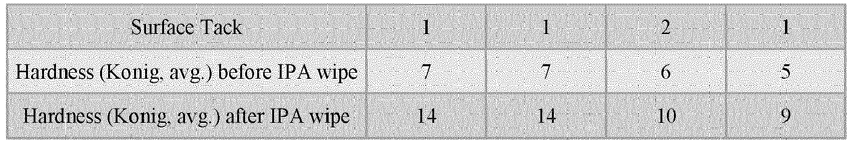

The performance properties of the coating compositions containing the acidic urethane acrylates (III) and (IV) at 12.5% and 37.5% loading were very similar. This is important because it allows more acidic urethane acrylate to be added without changing the performance properties of the composition. As the amount of acidic urethane acrylate III is increased, the initial color (APHA, 10 mm) and yellowness (b^{∗}) of the films went up slightly. Initial haze was still low for all compositions. Konig hardness was similar for all samples before and after IPA wipe and similar to previous compositions 1, 2, 3, and 4 according to the invention. The removability of the coating compositions was measured in both water solution (pH =5) and base solution (pH = 9) to determine if changing the amount of acidic urethane acrylate III and IV had an effect on the swellability of the coatings.

Figure 3 shows the Invention compositions 5, 6, 7, and 8 compared to the Control 1 composition. Invention compositions 7 and 8 did show excellent swellability and removability in base solution. Again, none of the compositions showed any swellability/removability in water solution (pH = 5).

For Invention compositions 5 and 6, no swelling was present. It is expected that more rigorous soaking conditions (e.g., a longer soak time, a higher temperature, and/or a higher pH) would achieve some swelling. The crosslink density is important because if the composition is too highly crosslinked, the remover will not be able to penetrate through the film and trigger removability via a swelling mechanism. In addition, there has to be a certain number of functional groups present in the film that can allow for enough swelling. The data show that 12.5% of lower MW acidic urethane acrylate III, which has a higher crosslinking density, did not allow for triggered removability after 20 minutes soaking in base solution. However, 12.5% of a higher MW acidic urethane acrylate IV, which has larger channels to allow for solvent penetration through the film due to its lower crosslink density, had much better swellability after 20 minutes soaking in base solution.

Within this specification, embodiments have been described in a way which enables a clear and concise specification to be written, but it is intended and will be appreciated that embodiments may be variously combined or separated without departing from the invention. For example, it will be appreciated that all preferred features described herein are applicable to all aspects of the invention described herein.

In some embodiments, the invention herein can be construed as excluding any element or process step that does not materially affect the basic and novel characteristics of the compositions and methods described herein. Additionally, in some embodiments, the invention can be construed as excluding any element or process step not specified herein.

Although the invention is illustrated and described herein with reference to specific embodiments, the invention is not intended to be limited to the details shown. Rather, various modifications may be made in the details within the scope and range of equivalents of the claims and without departing from the invention.

## Claims

1. A curable urethane (meth)acrylate oligomer having no free isocyanate groups at the terminal ends of the oligomer and formed by reacting, in the presence of a catalyst, at least the following:
(A) a first isocyanate reactive component bearing at least one (meth)acrylate group and at least one isocyanate reactive group;
(B) a polyisocyanate component; and
(C) a second isocyanate reactive component bearing at least one acidic group and two isocyanate reactive groups,
wherein the urethane (meth)acrylate has an acid content of at least 5×10⁻⁴ mol acid/g oligomer.

2. The urethane (meth)acrylate oligomer according to claim 1, wherein the component (C) is dimethyl propionic acid (DMPA).

3. The urethane (meth)acrylate oligomer according to claim 1 or claim 2, wherein the component (A) comprises a lactone (meth)acrylate selected from the group consisting of β-propiolactone (meth)acrylate, γ-butyrolactone (meth)acrylate, δ-valerolactone (meth)acrylate and ε-caprolactone (meth)acrylate), and preferably is caprolactone acrylate.

4. The urethane (meth)acrylate oligomer according to any one of claims 1 to 3, wherein the polyisocyanate is isophorone diisocyanate (IPDI).

5. The urethane (meth)acrylate oligomer according to any one of claims 1 to 4, wherein the oligomer is formed by reacting a base (D) with components (A), (B), and (C) or alternatively, subsequent to reaction of components (A), (B), and (C), in an amount such that a majority of the acid groups present in the oligomer are in a salt form instead of a free acid form.

6. The urethane (meth)acrylate oligomer according to any one of claims 1 to 5, wherein the oligomer is formed by reacting (E) a polyol with components (A), (B), and (C) and optionally (D) a base.

7. The urethane (meth)acrylate oligomer of any of Aspects 1-6, having the formula (II): 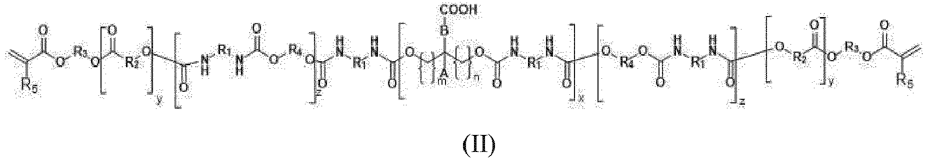 wherein:
each R₁, R₂, and R₃ is independently a C₁-C₁₂ aliphatic or cycloaliphatic linker;
R₄ is a residue of a polyol;
R₅ is either hydrogen or methyl;
A is hydrogen or a C₁-C₅ alkyl group;
B is a bond or a C₁-C₅ aliphatic linker; and
m and n are independently 1, 2, 3 or 4, with the proviso that the total number of carbon atoms in A, B, m, and n combined is an integer from 2 to 13; and
x and y are independently an integer from 0 to 5;
z is 0 or 1 to 10 and
optionally, at least a portion of the carboxylic acid groups exist in a salt form instead of a free acid form.

8. An ink or coating composition comprising the urethane (meth)acrylate oligomer according to any one of claims 1 to 7 and a photoinitiator, and at least one of a (meth)acrylate monomer or a (meth)acrylate oligomer and, optionally, at least one additive.

9. A method for making an article comprising:
applying the ink or coating composition according to claim 8 to a substrate; and
curing the ink or coating composition,
wherein the substrate is wood, plastic, metal, glass, stone, concrete, or a composite thereof, preferably plastic.

10. A method for recycling a substrate coated with the cured ink or coating composition ink according to the method of claim 9, wherein the method for recycling comprises:
contacting the article to which the cured ink or coating composition is attached with a recycling solution having a pH sufficient to delaminate the ink or coating composition from the substrate; and
retrieving from the recycling solution the substrate free from the ink or coating composition.

11. The method according to claim 10, wherein the recycling solution comprises a base selected from an inorganic base or an organic base.

12. A nail gel composition comprising:
the urethane (meth)acrylate oligomer according to any one of claims 1 to 7;
at least one of a (meth)acrylate monomer or a (meth)acrylate oligomer; and
optionally, at least one additive.

13. A method for coating a nail comprising:
applying the nail gel composition according to claim 12 to a nail; and
curing the nail gel composition.

14. A method for removing the cured nail gel composition coated on the nail according to the method of claim 13, wherein the method for removing the cured nail gel composition comprises:
immersing the cured nail gel composition coated on the nail with a soaking solution having a pH sufficient to partially or fully delaminate the cured nail gel composition from the nail without harming the nail;
wherein, if the immersing step only partially delaminates the cured nail composition from the nail thereby leaving a portion of the cured nail composition attached to the nail, then the method further comprises manually removing from the nail the portion of the cured nail composition attached to the nail while the nail is still present in the soaking solution or, alternatively, after the nail is removed from the soaking solution.

15. The method according to claim 14, wherein the pH of the soaking solution is ≥7 and less than 11, preferably less than 10, more preferably less than 9.5, and most preferably less than 9.
